# EUROPEAN PATENT APPLICATION

(11) **EP 1 295 561 A1**
(43) Date of publication of application: **26.03.2003**
(21) Application number: 02078750.3
(22) Date of filing: 11.09.2002
(51) Int. Cl.: A61B 10/00

(54) **Liquid specimen collection container.**

(30) Priority: 19.09.2001 US 323371
(71) Applicant: Becton, Dickinson and Company, Franklin Lakes, New Jersey 07417-1880 (US)
(72) Inventor: Szeles, Laszlo, Mountainside, New Jersey 07092 (US)
(74) Representative: Wittop Koning, Tom Hugo

(57) **Abstract**

A container assembly provides for the collection, transporting, and dispensing of a fluid specimen. The container assembly includes a cup-shaped container having an open end for collecting the fluid specimen. A lid is attachable to the container to close the open end thereof. The lid includes a receptacle for providing communication with the collected fluid specimen through the cover and for permitting extraction of a sample therefrom. A self-sealing closure member is supported by the lid in the receptacle and seals the receptacle preventing fluid leakage. The extraction device may be used to extract a sample of the fluid specimen. The extraction device is insertable into the receptacle to permit such extraction. Upon removal of the extraction device, the self-sealing closure reseals the container and prevents fluid leakage into the receptacle.

## Description

### BACKGROUND OF THE INVENTION

### 1. Field of the Invention

The present invention relates generally to a cup container used for collecting urine or other biological liquid specimens. An air-evacuated container may be used to extract portions of the specimen from the sealed container without removing the lid. The transfer may be done without pouring or pipetting.

### 2. Description of Related Art

In order to conduct laboratory testing on biological fluid samples such as urine, it is necessary to provide a container for collecting urine specimens. These specimen collection containers typically include a cup-shaped container with a removable cover. Once the sample has been collected in the container, the cover is reapplied. The specimen collection container may then be transported to a laboratory or other testing facility where a sample of the collected specimen is extracted for test purposes.

In order to simplify the sample extraction process, the prior art has seen the use of covers which not only cover and seal the collection container, but also provide for the use of an extraction device which permits the extraction of a sample of the fluid specimen. Such covers may include a sample port which supports a tube extending from the sample port to the lower end of the cup-shaped container in fluid communication with the specimen contained therein. The tube may include at its upper end a needle which extends to a location at the level of or above the cover so that at an air-evacuated collection container, such as a specimen collection tube, may be attached thereto to draw a portion of the collected sample thereinto without removal of the cover and removed without spilling or contaminating the sample port area. Subsequent samples may be drawn from the specimen collection container by using plural collection tubes.

Many of the prior art devices support the needle in the cover at the upper end of the collection container. The needle is generally exposed and may result in an accidental needle stick. Some prior art devices include a cap to cover the sample port opening or the needle tip itself to fully seal the specimen container and to prevent injury from an accidental needle stick when handling the sealed container. However, the caps must be removed in order to draw a sample of the specimen and replaced afterwards, exposing the user to risk of a needle stick at those times. Further, the needle contained in the cover is a separate metallic insert which must be disposed of as a sharps device.

It is, therefore, desirable to provide an improved specimen collection container which safely collects, transports and dispenses a fluid specimen and which can be easily and safely disposed of after use.

### SUMMARY OF THE INVENTION

According to the present invention, a container assembly for safely collecting, transporting and dispensing a fluid specimen, which can be easily and safely disposed of, e.g. by incineration, is provided.

In one aspect, the invention relates to a container assembly which includes a cup-shaped container having sidewalls joining a bottom wall and an opposed open end to define a container interior for collecting a fluid specimen, a lid attachable to the container to close the open end thereof, a cannula and a self-sealing closure member.

The lid has a central portion, a peripheral margin, a sealing flange at the periphery of the peripheral margin and a continuous, elongated receptacle having an opening located within the central portion for insertable receipt of an evacuated tube having a cannula pierceable stopper. The peripheral flange extends from the peripheral margin towards the bottom wall of the container and sealingly engages the open end of the container when the lid is sealed on the container. The elongated receptacle includes a lower wall extending from the central portion of the lid into the container interior and towards the bottom wall of the container when the lid is placed on the container.

The cannula is supported by the lower wall of the receptacle and has a first needle end positioned within the receptacle at a depth into the opening substantially below the sealing flange, in a position to pierce the stopper of an evacuated tube when the tube is received with its pierceable stopper end first into the receptacle, and a second needle end positioned within the container interior, when the lid is placed on the container, so that communication between the container and the tube is established when the tube is inserted in the receptacle.

The self-sealing closure member prevents fluid communication between the container and the receptacle, allowing communication between the container and the tube when the tube is inserted in the receptacle and being self-sealing to prevent fluid communication between the container and the receptacle when the tube is retracted from the receptacle.

In one embodiment, the bottom wall of the container has an inner convex shaped surface and an outer concave shaped surface. Preferably, the container is transparent. The container can also have a fill level indicator which identifies the maximum fill level for collecting a fluid specimen. The fill level indicator is located so that the fluid specimen will not exceed the capacity of the container when the container is initially filled to the fill level indicator and then the lid is placed on the container.

Preferably, the elongated receptacle is generally cylindrical for accommodating an evacuated collection tube. In one embodiment, the opening of the elongated receptacle is offset from the center of the central portion of the lid, towards the peripheral margin of the lid, and the lower wall of the receptacle extends toward the bottom wall of the container at a location offset from the center of the bottom wall, towards the sidewalls, when the lid is placed on the container.

Preferably, the lower wall of the elongated receptacle extends to a distance of less than about 2 cm from the bottom wall of the container when the lid is placed on the container and, more preferably, less then about 1 cm from the bottom wall of the container when the lid is placed on the container.

The cannula is preferably formed of a polymeric resin. The cannula is also preferably molded in place on the lower wall of the elongated receptacle. More preferably, the cannula is continuous with and part of the molded surface of the lower wall of the receptacle. Preferably, the second needle end of said cannula is flush with the lower wall of the elongated receptacle. While a polymeric integrally molded cannula is preferred, the present invention also contemplates use of a stainless cannula which may be bonded in place.

In an embodiment where the container includes a fill level indicator, the first needle end of the cannula is positioned at a location within the receptacle at a depth from the opening substantially below the fill level indicator when the lid is placed on the container.

The first needle end is preferably positioned at a location within the receptacle such that the ratio of the distance between the opening of the receptacle and the first needle end to the distance between the lower wall of the receptacle and the first needle end is at least about 3:1 and, more preferably, at least about 4:1.

The first needle end is also preferably positioned at a location within the receptacle at a distance of less than about 2.0 cm from the lower wall of the receptacle and, more preferably, less than about 1.5 cm from the lower wall of the receptacle.

The self-sealing closure member is preferably a cannula pierceable self-sealing sleeve covering the first needle end of the cannula to prevent fluid communication between the container and the receptacle. The sleeve is pierceable to allow communication between the container and an evacuated tube when the tube is inserted in the receptacle and is self-sealing to prevent fluid communication between the container and the receptacle when the tube is retracted from the receptacle.

The sleeve is preferably a unitary device molded from a flexible, elastomeric material capable of resealing after being pierced by the cannula.

An advantage of the present invention is that it allows a patient and/or an operator to safely collect, transport and dispense a fluid biological specimen and otherwise safely handle the container assembly, without risk of an accidental needle stick.

Another advantage of the present invention is that, once the lid is sealed on the container, the operator can transport and dispense a fluid specimen without risk of contaminating the fluid specimen or the dispensed fluid sample, or being contaminated by the biological specimen.

Yet another advantage, in the case of a polymeric molded cannula, is that the container assembly can be easily and safely disposed of after use, e.g., by incineration, without the need to separately dispose of biological sharps, e.g., a metal needle.

### BRIEF DESCRIPTION OF THE DRAWINGS

FIG. 1 is an isometric view of the container and lid of an embodiment of the present invention;

FIG. 2 is a cross-sectional side elevation of the container and lid of FIG. 1;

FIG. 3 is a view similar to FIG. 2 with an air-evacuated tube inserted in the receptacle and forced over the needle point.

FIG. 4 is a side elevation of a preferred embodiment container of the invention with a cross-sectional view of one side showing the receptacle.

FIG. 5 is an isometric view of the lid component employed in the preferred embodiment container of FIG. 4.

FIG. 6 is a cross-sectional, side elevation of the lid component shown in FIG.5.

FIG. 7 is a view similar to FIG. 6 with an air-evacuated tube inserted in the receptacle and forced over the needle point.

### DETAILED DESCRIPTION OF THE INVENTION

The present invention provides a specimen collection assembly for safely collecting, transporting and dispensing a fluid specimen and allows for samples to be drawn from the specimen safely and without contamination of the specimen.

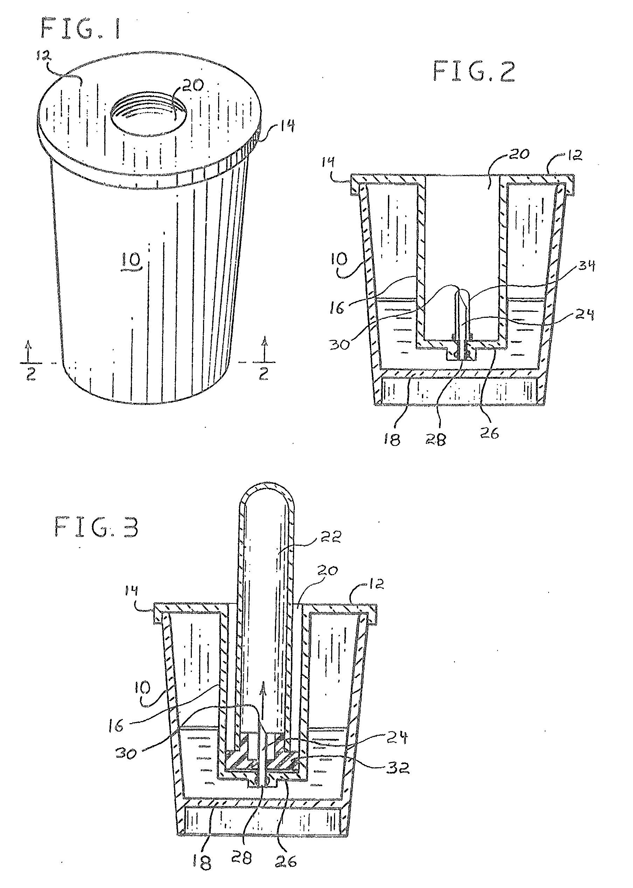

One embodiment of the invention provides a specimen collection container assembly that includes a cup for collecting liquid specimens and a lid having a receptacle and a cannula for use with an evacuated tube for withdrawing samples of the specimen. Referring to the drawings there is shown in FIGS. 1-3 a cup **10** in which urine or other biological liquid specimens may be collected after which the patient or supervising nurse places a lid **12** on the cup.

The lid **12** has a flange **14** around its outer rim of a size to provide a tight fit when the lid is placed over the cup **10**. The lid has an elongated receptacle **16** extending into the container towards the bottom wall of the cup **18** for insertable receipt of an evacuated tube having a cannula pierceable stopper. The receptacle **16** has an opening **20** for insertable receipt of an evacuated tube **22** and a cannula **24** passing through its lower wall **26** with the lower end of the cannula **28** flush with the lower wall **26** of the receptacle or extending further towards the bottom of the cup. The top end of the cannula projects through the lower wall of the receptacle so that its needle point **30** is adapted to pierce the stopper **32** of an air-evacuated tube **22** for withdrawing liquid from within the cup. The needle point of the cannula **30** is recessed within the receptacle **16** at a depth into the opening **20** sufficient to prevent injury to a patient or attendant when collecting a specimen, handling the container or withdrawing a sample of the specimen. The needle point of the cannula **30** is recessed within the receptacle at a depth from the opening **20** below the sealing flange **14**. The receptacle also has a self-sealing closure member **34** positioned to prevent fluid communication between the container interior and the receptacle. The self-sealing closure member **34** is preferably cannula pierceable to allow communication between the container interior and the evacuated tube when the tube is inserted in the receptacle and is self-sealing to prevent fluid communication between the container interior and the receptacle when the tube is retracted from the receptacle.

When a sample of the specimen is to be removed from the cup it is only necessary to place the stopper end of the air-evacuated tube **22** into the receptacle so that the needle pierces the stopper **32** of the tube **22**. Since it is not necessary to remove the lid **12** and the needle point remains isolated from the receptacle as a result of the self-sealing closure member, no outside contamination of the tube **22** occurs. Furthermore, following shaking or mixing of the specimen, liquid does not run down the outside of the cup as with most specimen cups requiring removal of the lid. If the air-evacuated tube **22** containing either a urine preservative or a sputum digestant is placed on the needle, there will be no contamination of the specimen container or tube **22** when it is sent to the laboratory. This method provides a means for reducing the possibility of contaminating the nurse and laboratory worker with infectious agents present in the specimen by eliminating the need for pouring or pipetting portions of the specimen to several containers for processing by various departments in the laboratory.

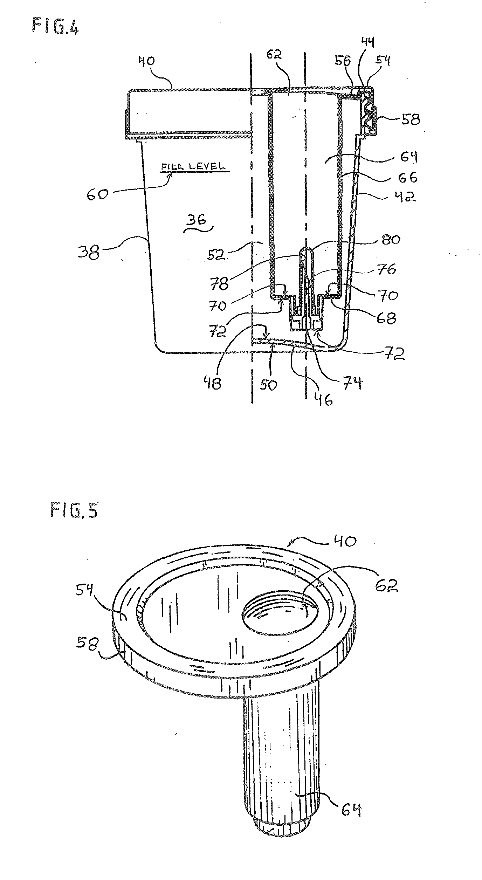

Those skilled in the art will appreciate that many variations of the above described embodiment of the invention may be made without departing from the spirit and the scope of the invention. For example, referring now to FIG. 4, there is seen a side elevation of a preferred embodiment collection container assembly **36** of the invention with a cross-sectional view of one side of the container assembly **36** which includes a receptacle for receiving an evacuated sample tube. The container assembly **36** comprises a cup **38** portion and a removable lid **40** portion. The cup **38** comprises a slightly tapering, tubular vessel having continuous, tapered sidewalls **42** separating an open end **44** and a closed end **46**. The closed end **46** has a convex shaped inner surface **48** and a concave shaped outer surface **50** to assist in maximum sample collection of small volume fluids in the bottom of the cup **38**. The lid **40** is removably attached on the open end **44** of cup **38**. The cup **38** with lid **40** together define a collection chamber **52** which is suitable for holding biologically hazardous materials. The cup **38** and lid **40** may be fashioned from any conventional material such as, for example, a polymeric resin. Polymeric resins are well known in the art and include for example polyethylene, polycarbonate, polystyrene and like polymeric resinous materials.

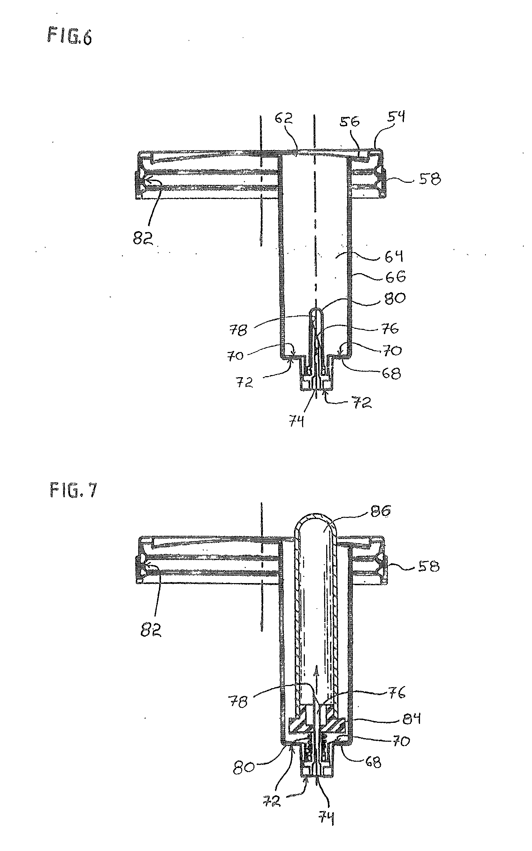

The lid **40** component is shown in an isometric view in FIG. 5 and comprises a generally disc shaped closure member having an outer peripheral zone **54** and an inner or central zone **56**. A skirt (or flange) **58** depends downwards from the outer peripheral zone **54** of the lid **40**. It will be observed in FIG. 5 that the flange **58** depends downward from peripheral zone **54** of the lid member **40** to partially hide the underside of lid **40**. The flange includes an inner surface which contains a means for sealingly engaging the lid with the open end of the cup. The side wall **42** of the cup **38** can also contain a fill level indicator **60** which identifies a maximum fill level for collecting a fluid specimen. The fill level indicator is positioned so that the fluid specimen will not exceed the capacity of the collection chamber **52** when the cup **38** is initially filled to the fill level indicator (i.e., before the lid is attached) and the lid **40** is attached to the cup **38**. Positioned at the central zone **56** is an opening **62** to an elongated tubular receptacle **64**, preferably offset towards the outer peripheral zone **52**. The receptacle is formed by sidewalls **66** and lower wall **68**, having an inner surface **70** and an outer surface **72**, which are continuous with and part of the molded surface of the lid component **40**. The receptacle **64** as defined by its sidewalls **66** and lower wall **68** projects inwardly into the chamber **52** of cup **38** and extends towards the bottom wall **46** of the cup **38**, when lid **40** is attached to close the open end **44** of cup **38**. The central portion of the lower wall **68** extends a further distance towards the bottom wall **46** of the cup **38** (when the lid **40** is attached to the cup **38**). The central portion of the outer surface **72** of the lower wall **68** is preferably adjacent to the inner surface **48** of the closed end **46** of the cup **38** and offset from the central portion of the closed end **46**, towards the sidewalls **42** of the cup **38**. The outer surface **72** of the lower wall **68** has an opening **74** to a tubular cannula **76** located at the central portion of the lower wall **68**.

The tubular cannula **76** is continuous with and part of the molded lower wall **68** of the receptacle **64.** The cannula **76** has a first end defined by the opening **74** in the central portion of the lower wall **68** of the receptacle **64** and a second needle point **78** end which projects from the inner surface **70** of the lower wall **68** into the receptacle **64** in a position to pierce the stopper of an evacuated tube when the tube is received with its pierceable stopper end first into the receptacle. The cannula is in open communication between chamber **52** and an evacuated tube when the tube is inserted in the receptacle and the stopper is pierced. The needle point **78** end of the cannula **76** is recessed within the receptacle **64** at a depth from the opening **62** sufficient to prevent injury to a patient or attendant when handling the container, collecting a specimen, transporting the specimen or withdrawing a sample of the specimen. The needle point **78** of the cannula **76** is preferably recessed within the receptacle at a depth from the opening **62** below the fill level indicator **60** on the sidewall **42** of the cup **38**, when the lid **40** is attached to close the open end **44** of cup **38**.

A cannula pierceable self-sealing sleeve **80** is secured over the cannula **76**, covering the second needle end **78** and preventing fluid communication between the chamber **52** and the receptacle **64**. The sleeve **80** is pierceable to allow communication between the chamber **52** and the evacuated tube when the tube is inserted in the receptacle **64** (and the stopper is pierced) and self-sealing to prevent fluid communication between the chamber **52** and the receptacle **64** when the tube is retracted from the receptacle. The sleeve **80** is preferably a unitary device molded of any flexible, elastomeric material conventionally used for fabricating gas-proof closures. Representative of such materials are natural rubber (cis-1,4-polyisoprene, molecular weight 100,000 to 1,000,000), polyurethane elastomers, butyl rubber (copolymers of isobutylene and diolefins) and the like. Preferred elastomeric materials are those of low gas permeability such as butyl rubbers having a Shore A hardness of circa 35 to 80.

This structure is particularly advantageous in that it allows the operator to safely handle the container assembly **36** and to insert an evacuated tubular container into the receptacle **64** in the same manner previously described in reference to the previously described embodiment container **10**. In this way, the operator can conveniently and safely withdraw biologically hazardous materials from chamber **52** into the inserted evacuated, tubular container. When a sufficient portion of biologically hazardous material has been withdrawn into the evacuated, tubular container from chamber **52**, via cannula **76**, the operator may remove the inserted tubular container from receptacle **64**, interrupting communication of cannula **76** from the tubular container and preventing communication with the receptacle **64**. Those skilled in the art will readily appreciate that the integral, unitary structure of the collection container **36** serves to protect the operator from an unnecessary exposure to potentially hazardous biological materials which may be contained within chamber **52**.

Referring now to FIGS. 6-7, further details of the lid component **40** may be observed. FIG. 6 is a side elevation of lid **40** in cross section and FIG. 7 is a similar view with an air-evacuated tube inserted in the receptacle and forced over the needle point of the cannula. The inner surface **82** of flange **58** and the outer surface of the sidewalls **42** adjacent to the open end **44** of the cup **38** can include mating threads or other means for temporarily and sealingly attaching the lid **40** to the cup **38**. Other means for temporarily and sealingly attaching the lid **40** may include mating ridges or protrusions extending in an annular fashion to provide a snap-on attachable lid. Other means for temporarily and sealingly attaching the lid **40** to the cup **38** are also contemplated.

The elongated tubular receptacle **64** projects inwardly into the chamber **52** of cup **38** when lid **40** is placed on the open end **44** of the cup **38** and extends to a point so that the outer surface **72** of its lower wall **68** is adjacent to the inner surface **48** of the closed end **46** of the cup **38**. The outer surface **72** of the lower wall **68** preferably extends to a distance of less than about 2 cm from the inner surface **48** of the closed end **46** of the cup **38** and, more preferably, less than about 1 cm from the inner surface **48** closed end **46** of the cup **38**, when the lid **40** is sealed on the open end **44** of the cup **38**.

The cannula **76** is continuous with an part of the molded lower wall **68** of the receptacle **64**. Preferably, the lid **40** is molded from a polymeric resin as one continuous piece, including the receptacle **64** and the cannula **76**. The needle point **78** of the cannula **76** is located within the receptacle **64** in a position to pierce the stopper **84** of an evacuated tube **86**, when the tube is received with its stopper end first into the receptacle, and at a sufficient distance from the opening **62** of the receptacle **64** to prevent injury to an operator from an accidental needle stick during handling. The needle point **78** of the cannula **76** is recessed within the receptacle at a depth from the opening **62** substantially below the sealing flange **58** of the lid **40**. This results in the needle point of the cannula **76** being located substantially below opening **62** of receptacle **64**. Preferably, the needle point **78** is positioned so that the ratio of the distance between the opening **62** of the receptacle **64** and the needle point **78** to the distance between the needle point **78** and the inner surface **70** of the lower wall **68** of the receptacle **64** is at least about 3:1 and, more preferably, at least about 4:1. The needle point **78** preferably projects from the inner surface **70** of the lower wall **68** of the receptacle **64** to a minimum distance sufficient to reliably and repeatedly pierce the stopper **84** of an evacuated tube **86** and establish fluid communication between the chamber **52** of the cup **38** and the tube **86**, when the tube is received with its stopper end first into the receptacle. The needle point **78** preferably projects from the outer or peripheral portion of the inner surface **70** of the lower wall **68** of the receptacle **64** to a distance of less than about 2.0 cm from the inner surface **70**, more preferably, less than about 1.5 cm, and, most preferably, about 1.0 cm.

The cannula pierceable self-sealing sleeve **80** is secured over the cannula **76** and prevents fluid communication between the cannula **76** and the receptacle **64.** The sleeve **80** is pierceable to allow communication between the cannula opening **74** and the evacuated tube **86**, when the tube is inserted into the receptacle, stopper end first, and the stopper **84** is pierced by the needle end **78** of the cannula **76**. The sleeve **80** is made from a resilient flexible material which allows the sleeve **80** to be pierced and pushed down over the cannula **76** by the stopper **84** of the tube **86**, as shown in FIG. 7, and allows the sleeve **80** to restore itself, to its original position when the tube is retracted and to reseal, preventing fluid communication between the cannula **76** and the receptacle **64**, as shown in FIG. 6.

The container **10** or **36** according to the invention is intended to be used in the first instance by a patient, and then by a doctor, nurse or laboratory technician in the second instance for sampling of the collected specimen. The patient uses the container **10** or **36** by removing the entire cover assembly **12** or **40** and then providing the sample. The cover **12** or **40** is then reapplied by the patient, and the container **10** or **36** is given to the test person. That person inserts the evacuated specimen vial 22 or 86 into the receptacle **16** or **84** stopper end first until the needle point **30** or **78** pierces the pierceable stopper end. The vacuum of the vial **22** or **86** then causes a portion of the sample to be drawn up the cannula **24** or **76** into the vial **22** or **86**. The vial **22** or **86** can then be withdrawn or retracted from the receptacle **16** or **64** and a second, third, etc. vial inserted and forced over the needle point **30** or **78** to withdraw additional portions of the sample. Once the final desired portion is withdrawn, the container **10** or **36** is then discarded or further handled as desired.

Those skilled in the art will appreciate the simple, unitary construction of the container **10** or **36** and the ease with and safety which it may be operated in association with air-evacuated, tubular containers of the type described in conjunction with the container embodiment **10** or **36**. The protection that the containers **10** and **36** offer to the operator are evident. There are no loose parts in the container assembly which require separate manipulation for the transfer of contained materials and the container reseals itself upon withdrawing the evacuated sample tube.

## Claims

1. A container assembly for collecting, transporting and dispensing a fluid specimen comprising:
a cup-shaped container having sidewalls joining a bottom wall and an opposed open end define a container interior for collecting said fluid specimen;
a lid attachable to said container to close said open end thereof, said lid having a central portion, a peripheral margin, a sealing flange at the periphery of the peripheral margin and a continuous, elongated receptacle having an opening located within the central portion for insertable receipt of an evacuated tube having a cannula pierceable stopper, said peripheral flange extending from the peripheral margin towards the bottom wall of the container and making sealing engagement with the open end of the container when the lid is sealed on the container, said elongated receptacle including a lower wall extending from the central portion of the lid into the container interior and towards the bottom wall of the container when the lid is placed on the container;
a cannula supported by said lower wall of said receptacle with a first needle end positioned within the receptacle at a depth into said opening substantially below the sealing flange, in a position to pierce the stopper of an evacuated tube when said tube is received with its pierceable stopper end first into said receptacle, and a second needle end within the container interior so that communication between said container and said tube is established when said tube is inserted in the receptacle; and
a self-sealing closure member preventing fluid communication between the container and the receptacle, said closure member allowing communication between the container and the tube when the tube is inserted in the receptacle and being self-sealing to prevent fluid communication between the container and the receptacle when the tube is retracted from the receptacle.

2. A container assembly of claim 1 wherein said elongated receptacle is generally cylindrical for accommodating said evacuated collection tubes.

3. A container assembly of claim 1, wherein said cannula is formed of a polymeric resin.

4. A container assembly of claim 3, wherein said plastic cannula is molded in place on the lower wall of said elongated receptacle.

5. A container assembly of claim 1, wherein said lid is screw attachable to said container.

6. A container assembly of claim 1, wherein said lid is snap-on attachable to said container.

7. A container assembly of claim 1, wherein the bottom wall of said container comprises an inner convex shaped surface and an outer concave shaped surface.

8. A container assembly of claim 7, wherein the opening of said elongated receptacle is offset from the center of the central portion of said lid, towards the peripheral margin of said lid, and the lower wall of said receptacle extends towards the bottom wall of the container at a location offset from the center of the said bottom wall, towards the sidewalls, when the lid is placed on the container.

9. A container assembly of claim 8, wherein the second needle end of said cannula is flush with the lower wall of said elongated receptacle.

10. A container assembly of claim 1, wherein the lower wall of said elongated receptacle extends to a distance of less than 2 cm from the bottom wall of said container when the lid is placed on the container.

11. A container assembly of claim 10, wherein the lower wall of said elongated receptacle extends to a distance of less then 1 cm from the bottom wall of said container when the lid is placed on the container.

12. A container assembly of claim 1, wherein the first needle end is positioned at a location within the receptacle such that the ratio of the distance from the opening of said receptacle to said first needle end to the distance from the lower wall of said receptacle to said first needle end is at least about 3:1.

13. A container assembly of claim 12, wherein said ratio is at least about 4:1.

14. A container assembly of claim 1, wherein the first needle end is positioned at a location within the receptacle at a distance of less than about 2.0 cm from the lower wall of said receptacle.

15. A container assembly of claim 14, wherein the first needle end is positioned at a distance of less than about 1.5 cm from the lower wall of said receptacle.

16. A container assembly of claim 1, wherein said container is transparent.

17. A container assembly of claim 16, wherein said container further comprises a side wall having a fill level indicator which identifies the maximum fill level for collecting said fluid specimen.

18. A container assembly of claim 17, wherein said fill level indicator is located so that the fluid specimen will not exceed the capacity of said container when said container is initially filled to the fill level indicator and the lid is placed on the container.

19. A container assembly of claim 18, wherein said first needle end is positioned at a location within the receptacle at a depth from the opening substantially below said fill level indicator when the lid is placed on the container.

20. A container assembly of claim 1, wherein said self-sealing closure member comprises a cannula pierceable self-sealing sleeve covering the first needle end of said cannula to prevent fluid communication between said container and said receptacle.

21. A container assembly of claim 20, wherein said sleeve is pierceable to allow communication between said container and an evacuated tube when the tube is inserted in the receptacle and is self-sealing to prevent fluid communication between said container and said receptacle when the tube is retracted from the receptacle.

22. A container assembly of claim 21, wherein said sleeve comprises a unitary device molded from a flexible, elastomeric material capable of resealing after being pierced by said cannula.

23. A container assembly for collecting, transporting and dispensing a fluid specimen comprising:
a cup-shaped container having an open end defining a container interior for collecting said fluid specimen;
a lid attachable to said container to close said open end thereof, said lid having a continuous, elongated receptacle having an opening located within the central portion for insertable receipt of an evacuated tube having a cannula pierceable stopper, said elongated receptacle including a lower wall adjacent the bottom wall when the lid is placed on the container;
a cannula supported by said lower wall of said receptacle with a first needle end positioned within the receptacle at a depth into said opening substantially below said opening, in a position to pierce the stopper of an evacuated tube when said tube is received with its pierceable stopper end first into said receptacle, and a second needle end within the container interior so that communication between said container and said tube is established when said tube is inserted in the receptacle; and
a self-sealing closure member preventing fluid communication between the container and the receptacle, said closure member allowing communication between the container and the tube when the tube is inserted in the receptacle and being self-sealing to prevent fluid communication between the container and the receptacle when the tube is retracted from the receptacle.
